# EUROPEAN PATENT SPECIFICATION

(11) **EP 3 734 278 B1**
(45) Date of publication and mention of the grant of the patent: **06.07.2022**
(21) Application number: 18896558.6
(22) Date of filing: 27.12.2018
(51) Int. Cl.: G01N 33/53, G01N 33/48, G01N 33/569

(54) **INFORMATION ACQUISITION SYSTEM**
INFORMATIONSERFASSUNGSSYSTEM
SYSTÈME D'ACQUISITION D'INFORMATIONS

(30) Priority: 27.12.2017 WO PCT/JP2017/047032
(43) Date of publication of application: 04.11.2020
(73) Proprietor: KONICA MINOLTA, INC., Tokyo 100-7015 (JP)
(72) Inventor: SAITO, Haruyuki, Tokyo 100-7015 (JP); FUTAYA, Etsuko, Tokyo 100-7015 (JP)
(74) Representative: MFG Patentanwälte Meyer-Wildhagen Meggle-Freund Gerhard PartG mbB
(86) International application number: PCT/JP2018/048209
(87) International publication number: WO 2019/131895

(56) References cited:
- WO-A1-2017/181176
- US-A1- 2003 073 149
- NAING YE AUNG ET AL: "Specific Neuropilins Expression in Alveolar Macrophages among Tissue-Specific Macrophages", PLOS ONE, vol. 11, no. 2, 22 February 2016 (2016-02-22), pages 1-18, XP055623812, DOI: 10.1371/journal.pone.0147358
- KEIGO KUBOTA ET AL: "CD163+CD204+ tumor-associated macrophages contribute to T cell regulation via interleukin-10 and PD-L1 production in oral squamous cell carcinoma", SCIENTIFIC REPORTS, vol. 7, no. 1755, 11 May 2017 (2017-05-11) , pages 1-12, XP055623818, DOI: 10.1038/s41598-017-01661-z
- YOICHIRO KAKU ET AL: "Overexpression of CD163, CD204 and CD206 on Alveolar Macrophages in the Lungs of Patients with Severe Chronic Obstructive Pulmonary Disease", PLOS ONE, vol. 9, no. 1, 30 January 2014 (2014-01-30), pages 1-8, XP055622910, DOI: 10.1371/journal.pone.0087400
- TAKAHIRO TSUJIKAWA ET AL: "Quantitative Multiplex Immunohistochemistry Reveals Myeloid-Inflamed Tumor-Immune Complexity Associated with Poor Prognosis", CELL REPORTS, vol. 19, no. 1, 4 April 2017 (2017-04-04), pages 203-217, XP055623821, US ISSN: 2211-1247, DOI: 10.1016/j.celrep.2017.03.037
- AUNG, N. Y. et al.: "Specific Neuropilins Expression in Alveolar Macrophages among Tissue-Specific Macrophages", PLOS ONE, vol. 11, no. 2, e147358, 22 February 2016 (2016-02-22), pages 1-18, XP055623812,
- KUBOTA, K. et al.: "CD 163+ CD 204+ tumor-associated macrophages contribute to T cell regulation via interleukin-10 and PD-L1 production in oral squamous cell carcinoma", Scientific Reports, vol. 7, no. 1755, 11 May 2017 (2017-05-11) , pages 1-12, XP055623818,
- KAKU, Y. et al.: "Overexpression of CD 163, CD 204 and CD 206 on Alveolar Macrophages in the Lungs of Patients with Severe chronic obstructive pulmonary disease", PLOS ONE, vol. 9, no. 1, 30 January 2014 (2014-01-30), pages 1-8, XP055622910,
- TSUJIKAWA, T. et al.: "Quantitative Multiplex Immunohistochemistry Reveals Myeloid-Inflamed Tumor-Immune Complexity Associated with Poor Prognosis", Cell Reports, vol. 19, no. 1, 4 April 2017 (2017-04-04), pages 203-217, XP055623821,
- CANNARILE, M. A. et al.: "Targeting tumor-associated macrophages in cancer therapy and understanding their complexity", Oncoimmunology, vol. 3, no. 9, October 2014 (2014-10), - 8 December 2014 (2014-12-08), pages 1-2, XP055338112,
- ESCRIBESE, M. M. et al.: "The Prolyl Hydroxylase PHD3 Identifies Proinflammatory Macrophages and Its Expression Is Regulated by Activin A", The Journal of Immunology, vol. 189, no. 4, 15 August 2012 (2012-08-15), pages 1946-1954, XP055622906,
- ZHONG, W.Q. et al.: "M2-polarized macrophages in keratocystic odontogenic tumor: relation to tumor angiogenesis", Sientific Reports, vol. 5, no. 15586, 28 October 2015 (2015-10-28), pages 1-11, XP055622907,
- RIES, C. H. et al.: "Targeting Tumor-Associated Macrophages with Anti-CSF-1R Antibody Reveals a Strategy for Cancer Thera", Cancer Cell, vol. 25, 2014, pages 846-859, XP028855510, DOI: doi:10.1016/j.ccr.2014.05.016
- KOH, Y. W. et al.: "CSF-1R Expression in Tumor-Associated Macrophages Is Associated With Worse Prognosis in Classical Ho", American Journal of Clinical Pathology, vol. 141, 2014, pages 573-583, XP055335369, DOI: doi:10.1309/AJCPR92TDDFARISU

## Description

### Technical Field

The present invention relates to an an immunostaining method.

### Background Art

Cancer is a disease dividing, together with a vascular disease represented by myocardial infarction and cerebral infarction, the cause of death in adults in two. For example, the morbidity of breast cancer is lower in Japan than in Western countries, but has been increasing in recent years. In 1998, the morbidity of breast cancer surpassed the morbidity of gastric cancer and became the highest morbidity in women. According to a recent report by the Ministry of Health, Labor and Welfare in 2005, the annual number of breast cancer patients exceeded 50,000. Similarly, the number thereof is increasing every year in the world. According to a report by WHO in 2008, the morbidity of breast cancer was the highest in men and women, and the annual number of breast cancer patients exceeded 1.38 million, which accounted for about 23% of all cancers in women.

The microenvironment surrounding cancer cells has a great effect on the growth of cancer, and in particular, the immune system environment of tumor tissues has recently attracted attention under the keyword "cancer immunity". In the immune system, various immune cells such as macrophages, dendritic cells, B cells, T cells (helper T cells, killer T cells, inhibitory T cells) and natural killer cells are working. Among them, macrophages are particularly important immune cells.

Macrophages are important cells for formation of the microenvironment of cancer together with fibroblasts, vascular endothelial cells and the like, and it is known that many macrophages are present around cancer cells. Macrophages are divided into two phenotypes whose physiological roles are completely different from each other, called pro-inflammatory M1 type (hereinafter, referred to as M1 macrophage) and anti-inflammatory M2 type (hereinafter, referred to as M2 macrophage) (Non Patent Literature 1). Macrophages infiltrating a tumor tissue are called tumor-associated macrophages (TAMs).

It is known that TAMs mainly consist of an M2 macrophage population (Non Patent Literature 2). Also, it is known that TAMs effectively suppress T cell activity and regulate signal transduction to promote cell proliferation and metastasis of cancer (Non Patent Literature 3). Clinical studies have also revealed a relation between the status of TAM and the poor prognosis of human tumors, and TAMs are currently considered a promising target for tumor therapy (Non Patent Literature 4).

In Non Patent Literature 5, a target protein in tumor therapy targeting TAMs, macrophage protein CSF-1R (colony stimulating factor 1 receptor/MCSFR) is focused, and stained by immunostaining using the chromogenic substrate DAB.

However, in the case of staining with an enzyme such as DAB staining, since the stained density is greatly affected by environmental conditions such as temperature and time, it is unfortunately difficult to accurately estimate the actual amount of an antigen or the like from the stained density. Furthermore, in the Literature in which CD163 and CD68 are stained as M2 macrophage markers, the staining is performed on a tissue section different from that subjected to CSF-1R staining. Accordingly, it is not clear whether the detected CSF-1R is indeed expressed in macrophages.

Patent Literature 1 describes a method for performing immunostaining of a target protein using fluorophore-accumulated particles capable of showing with high accuracy the number of molecules and position of protein as luminescent spots having high luminance

Non Patent Literature 6 describes three-fold immunohistochemistry staining of macrophages using non-fluorescent dyes.

### Citation List

### Patent Literature

Patent Literature 1: WO 2013/035703

### Non Patent literature

Non Patent Literature 1: Mantovani A et al., Trends Immunol, 2004; 23: 549-55.
Non Patent Literature 2: Mills et al., J. Immunol. 164, 2000, p. 6166-6173
Non Patent Literature 3: Mantovaniet al., Novartis. Found. Symp., 256, 2004, p. 137-145
Non Patent Literature 4: Weigert Aet al., Immunotherapy. 2009 Jan; 1(1): 83-95.
Non Patent Literature 5: Cancer Cell 25, 846-859, June 16, 2014

Non Patent Literature 6: Aung et al., PLOS ONE, 2016; 11: 1-18.

### Summary of Invention

### Technical Problem

An object of the present invention is to acquire information on a target protein in a sample by detecting the target protein in an immune cell selected from a macrophage, T cell, natural killer cell, dendritic cell, B cell, granulocyte and plasma cell with high accuracy.

### Solution to Problem

The present inventors have found that a target protein expressed in an immune cell can be detected with higher accuracy by staining an immune cell protein and a target protein on the same section.

In other words, the present invention provides an immunostaining method as defined in appended claim 1.

### Advantageous Effects of Invention

According to the immunostaining method of the present invention, it is possible to acquire information on a target protein in a sample by detecting the target protein in an immune cell selected from a macrophage, T cell, natural killer cell, dendritic cell, B cell, granulocyte and plasma cell with high accuracy.

### Brief Description of Drawings

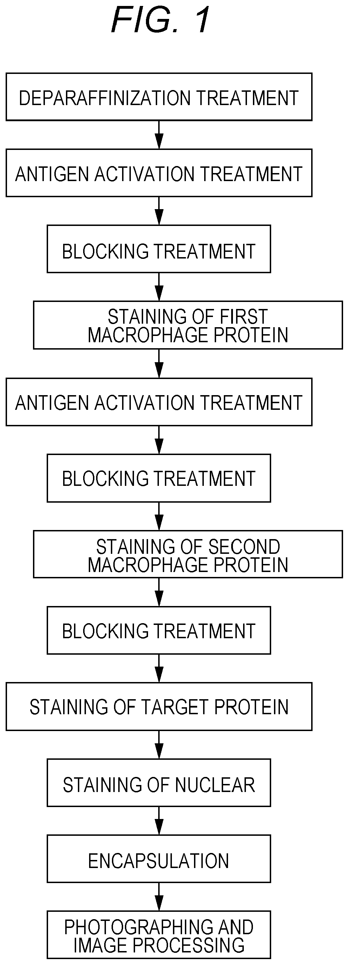

Fig. 1 is a flowchart illustrating an example of an immunostaining method.

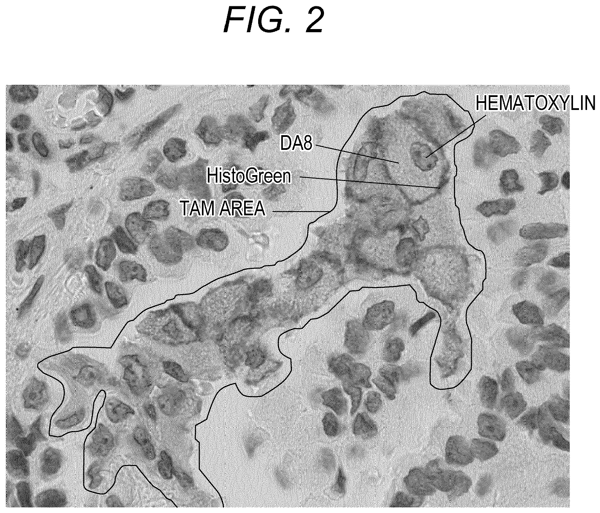

Fig. 2 is a dye-stained image photographed in Example 1.

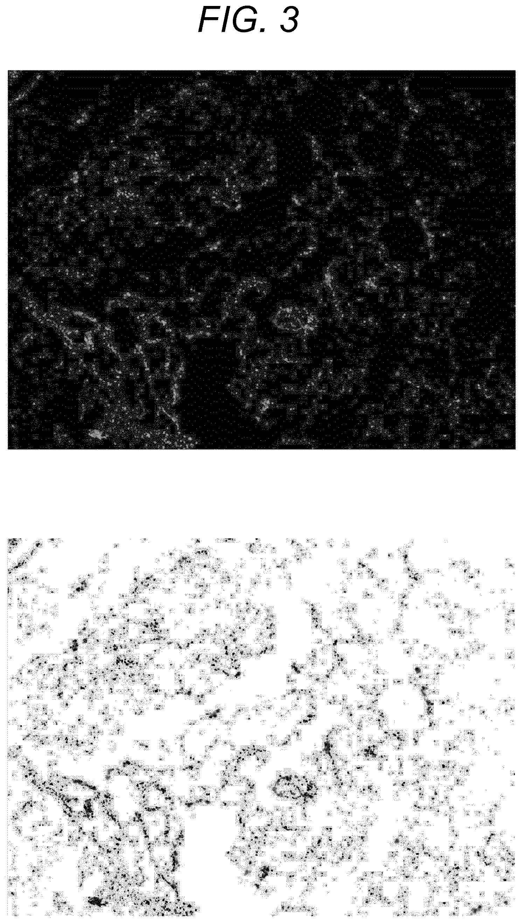

Fig. 3 shows a fluorescent image (upper; white indicates a luminescent spot) photographed in Example 1 and an image (lower) obtained by inverting the fluorescent image.

### Description

Hereinafter, a detailed description is made of an immunostaining method.

As one aspect the immunostaining method of the present invention comprises the following step (A) to (D): a step (A) of staining a first immune cell protein expressed in one or more cells selected from macrophages, T cells, natural killer cells, dendritic cells, B cells, granulocytes and plasma cells; a step (B) of staining a second immune cell protein expressed in one or more cells selected from macrophages, T cells, natural killer cells, dendritic cells, B cells, granulocytes and plasma cells; a step (C) of staining a target protein; and a step (D) of, after the steps (A) to (C), measuring a signal derived from the target protein,wherein the steps (A) to (C) are performed on a same sample, the sample being a tissue section collected from a subject or cells obtained by culturing a cell contained in a tissue collected from a subject, and the target protein being at least one protein expressed in the sample, and wherein the staining in the steps (A) and (B) is dye-immunostaining,the staining in the step (C) is fluorescent-immunostaining, and the fluorescent-immunostaining in the step (C) is performed after the steps (A) and (B).

According to the present invention, what is stained in the step (A) or step (B) is a first and a second immune cell protein expressed in immune cells (immune cell protein). The protein is an immune cell protein expressed in one or more cells selected from cells involved in immunity, i. e. macrophages, T cells such as helper T cells, killer T cells and regulatory T cells, natural killer cells, dendritic cells, B cells, granulocytes and plasma cells. In other words, here, the immune cell protein may include a macrophage protein.

Accordingly, in one aspect of the present invention, a step of staining a first immune cell protein (A), a step of staining a second immune cell protein (B), a step of staining a target protein (C), a step of measuring a signal derived from the target protein (D) are included. The steps (A) to (C) are steps performed on the same sample.

In the immunostaining method of the present invention, the staining in the steps (A) and (B) is dye-immunostaining, the staining in the step (C) is fluorescent-immunostaining, and the fluorescent-immunostaining in the step (C) is performed after the steps (A) and (B).

In the mmunostaining method of the present invention, it is preferable to further include a step (E) in addition to the steps (A) to (D), more preferable to include steps (E) and (F). Here, the step (E) is a step of identifying the position and number of an immune cell by staining in the step (A) and the step (B), preferably a step of identifying the position and number of M2 macrophages. The step (F) is a step of identifying information on the expression state of the target protein based on the signal derived from the target protein measured in the step (D) and the position and number of macrophages identified in the step (E).

The "information on the expression state of the target protein" is not particularly limited, but includes a histogram or a curve represented by the position where the target protein is expressed in a tissue or cells, the expression amount of target protein per cell or per unit area of tissue, or the expression amount of target protein per cell and the corresponding number of cells.

The information that can be obtained preferably include information based on the signal derived from the target protein measured in the step (D), more preferably information based on the information on the position and number of the immune cells identified in step (E), and the expression state of the target protein identified in the step (F).

Such information is not particularly limited, but examples thereof include the expression amount of target protein per unit area of a sample, for example the number of macrophages per unit area of a sample, the ratio of TAMs to the number of all macrophages contained in a sample, the expression amount of target protein in tumor cells and the expression amount thereof in macrophages (TAMs) per unit area of a sample, and their proportion, and the morphology of tissues or cells contained in a sample, preferably at least one of the position and expression amount of target protein, in particular in macrophages, more preferably the position and expression amount thereof.

The staining performed in the steps (A) and (B) is dye-immunostaining and the staining performed in the step (C) is fluorescent-immunostaining. The fluorescent-immunostaining in the step (C) is performed after the steps (A) and (B).

In addition, the "signal derived from the target protein" is preferably based on the number of luminescent spots derived from the fluorescently stained target protein.

The staining performed in the steps (A) to (C) is preferably performed by directly or indirectly binding a labeling substance to an immune cell protein and a target protein to be stained by contacting a sample described below with the labeling substance, and the example of the staining is preferably immunostaining performed by reacting a labeling antibody having a labeling substance bound to an antibody capable of directly or indirectly binding to the immune cell protein or a target protein with a sample.

As one aspect , when the dye-staining described below is performed in the step of staining a first macrophage protein (A) and the step of staining a second macrophage protein (B), cells can be identified from a variety of cell tumors or from several types of macrophages by labeling the desired macrophage protein with a dye. Such cell identification can be achieved by observing the dye used in the steps (A) and (B) under a bright field of view or by analyzing a bright field image.

For example, when performing the staining using DAB in the step (A) and using HistGreen in the step (B), cells stained with both DAB (brown) and HistGreen (green) can be identified as TAMs. In other words, it is possible to identify cells only by analyzing a bright field image (prior to signal measurement of fluorescent-staining of a target protein described below).

The cell identification also includes identifying cells as specific cells, for example, TAMs, by observing the coloring degree (coloring density) of the dye. In addition, examples of the cell identification in addition to identification of macrophages include identification of T cell types, identification of the type and cell cycle of cancer cells, identification of apoptotic/necrotic cells, and identification of dead cells/living cells/normal cells/cancer cells/cancer stem cells. Such cell identification is not limited to one type of identification, but a plurality of identification can also be performed at the same time. Furthermore, more information can be obtained by grasping the positional relationship between cells identified by bright field image analysis or the like.

### <Macrophage protein>

At least one of the first immune cell protein stained in the step (A) and the second immune cell protein stained in the step (B) is preferably a protein specifically expressed in M2 macrophages, also preferably a protein expressed in tumor-associated macrophages (TAMs). The first macrophage protein and the second macrophage protein are not particularly limited as long as they are proteins by which targeted macrophages, M2 macrophages or TAMs can be identified, but can be arbitrarily selected from proteins specifically expressed in macrophages.

Proteins specifically expressed in macrophages include CD163, CD204, CD68, Iba1, CD11c, CD206, CD80, CD86, CD163, CD181, CD197, iNOS, Arginase1, CD38 and Egr2.

In addition, proteins specifically expressed in M2 macrophages or TAMs include CD163, CD204 and CD206.

According to the present invention, what is stained in the step (A) or the step (B) is a protein expressed in immune cells (immune cell protein). The immune cell protein is expressed in one or more cells selected from cells involved in immunity, i. e. macrophages, and/or T cells such as helper T cells, killer T cells and regulatory T cells, dendritic cells, B cells, granulocytes and plasma cells.

CD4 (helper T cells), CD8 (killer T cells), CD16 or CD56 (natural killer cells), CD25 (IL-2R1, Tac or p55) or FoxP3 (regulatory T cells) are included.

### (Dye-immunostaining)

In the steps (A) and (B), dye-immunostaining is performed on the first immune cell protein and the second immune cell protein.

When the dye-immunostaining performed in the step (A) and the step (B), the cell identification , can be achieved.

Here, the dye-immunostaining not particularly limited as long as it is a technique of staining each immune cell protein with a bright field-observable dye. For example, widely used is a method for staining a target substance by depositing a dye on a sample by binding a labeling substance (enzyme) to a macrophage protein to be stained using any method, and adding a dye (substrate) that is colored through enzyme-substrate reaction. The immunostaining method of the invention includes adding, to a sample which has been previously reacted with a labeling antibody having the enzyme bound to an antibody capable of directly or indirectly binding to the target protein, a dye that is a substrate for the enzyme. The enzyme includes peroxidase and alkaline phosphatase, and the dye includes 3,3'-diaminobenzidine (DAB), Histogreen, TMB, Betazoid DAB, Cardassian DAB, Bajoran Purple, Vina Green, Romulin AEC, Ferangi Blue, Vulcan Fast Red and Warp Red.

When the first immune cell protein stained in the step (A) and the second immune cell protein stained in the step (B) are different from each other, the dyes used in the respective steps are preferably different dyes having a different hue, and also when the first immune cell protein stained in the step (A) and the second immune cell protein stained in the step (B) are the same as each other, the same dye is preferably used in the respective steps.

When dye-immunostaining using DAB is performed in either the step (A) or the step (B), it is preferable to firstly perform the step in which DAB is used from the viewpoint of dye stability.

For example, as to dye-immunostaining when performing tissue staining using a tissue array slide in the Examples described below, in the case where DAB and HistoGreen are used as dyes, when a first macrophage protein (CD68) is firstly stained with DAB, and then a second macrophage protein (CD 163) is stained with HistoGreen, there is no problem for color development of both DAB and HistGreen. As a result, in this Example, cells stained with CD68 and CD163 can be accurately judged as TAMs. However, when the order of DAB and HistoGreen was changed (for example, when CD 163 was stained with HistoGreen and then CD68 was stained with DAB), it was observed that color development of HistoGreen was poor. This is considered to be because HistoGreen cannot withstand the activation performed after the first staining so that the dye is dropped out.

### <Target protein>

The target protein stained in the step (C) in the information acquisition system of the present invention is at least one, preferably two or more proteins contained in a sample, preferably a tumor tissue. The target protein is not particularly limited, but examples thereof include receptors for colony stimulating factors such as CSF-1R, proteins that can be used as biomarkers in pathological diagnosis related to cancer such as PD-1, PD-L1 (Programmed cell death ligand 1), B7-1/2, CD8, CD30, CD48 and CD59, proteins involved in immune cell metabolism such as IDO (indoleamine oxygenase: indoleamine-2,3-dioxygenase-1), receptors for growth factors such as EGFR (HER1), HER2, HER3, HER4, VEGFR, IGFR and HGFR, and receptors for cytokines and chemokines such as CXCR2.

Specific target proteins are preferably CSF-1R, IDO, PD-1, PD-L1, B7-1/2, CD8, CD30, CD48, CD59 or CXCR2, particularly preferably CSF-1R, IDO, CXCR2, PD-1 or PD-L1.

The target protein is preferably a protein (antigen) particularly expressed in macrophages, more preferably a protein specifically expressed in macrophages, particularly preferably a protein specifically expressed in M2 macrophages. When a tumor tissue is used as the sample, the target protein is preferably a protein expressed in TAMs, more preferably a protein specifically expressed in TAMs.

The target protein that is a protein specifically expressed in TAMs is preferably CSF-1R, IDO, PD-L1, B7-1/2, CD8, CD30, CD48, CD59, or CXCR2, particularly preferably CSF-1R, IDO, CXCR2 or PD-L1.

In addition, when two or more of the target proteins are used, one of them is preferably CSF-1R. In this case, a protein to be targeted in tumor therapy is preferably used as the target protein, specifically a protein having an enhanced T cell response, an antitumor effect and the like is preferably used as the target protein in combination with CSF-1R. Specific examples thereof include IDO, PD-L1, PD-1 and CXCR2.

### (Fluorescent-immunostaining)

The staining of the target protein in the step (C) is fluorescent-immunostaining In this the steps (A) and (B) are dye-immunostaining, the fluorescent-imunostaining the step (C) is performed after the steps (A) and (B). By performing fluorescent-immunostaining after dye-immunostaining, it is possible to prevent a decrease in quantitativity due to deposition of a dye on a fluorescent substance.

For example, when performing tissue staining using a tissue array slide in the Examples described below, as described above, it is preferable that, as dye-immunostaining, staining with DAB precede that with HitoGreen. However, there may be a problem in that when, after DAB staining, fluorescent-immunostaining (staining of the target protein) is further performed before HitoGreen staining, the quantitativity of the target protein decreases.

Possible causes of the decrease in quantitativity of the target protein include dropout or deterioration of the fluorescent substance during activation in HistoGreen staining, or deposition of the dye on the fluorescent substance in HistoGreen staining.

Described herein are aspects for staining (i) one macrophage protein and one target protein, (ii) two macrophage proteins and one target protein, (iii) one macrophage protein and two or more target proteins, and (iv) two macrophage proteins and two or more target proteins are involved.

In performing aspect (i), for example, it is preferable that the first macrophage protein (for example, CD68) be firstly stained with DAB, and then the target protein (for example, PD-L1) be subjected to fluorescent-staining with a desired fluorescent dye. In addition, in performing aspect (ii), for example, it is preferable that the first macrophage protein (for example, CD68) be firstly stained with DAB, then the second macrophage protein (for example, CD 163) be stained with HistoGreen, and subsequently the target protein (for example, PD-L1) be subjected to fluorescent-staining with a desired fluorescent dye.

In addition, in performing aspect (iii), for example, it is preferable that the macrophage protein (for example, CD68) be firstly stained with DAB, and the target protein to be detected be subjected to fluorescent-staining with two or more desired fluorescent dyes. In this case, the fluorescent dyes for staining the respective target proteins are apart from each other preferably in emission wavelength such that there is no problem in fluorescence observation described later, more preferably by 40 nm to 150 nm.

Similarly, also in performing aspect (iv), for example, it is preferable that the first macrophage protein (for example, CD68) be firstly stained with DAB, then the second macrophage protein (for example, CD 163) be stained with HistoGreen, and subsequently the target protein to be detected be subjected to fluorescent-staining with two or more desired fluorescent dyes.

As described above, when performing fluorescent-staining under the aspects (ii) and (iv), the fluorescent-staining for the respective target proteins may be performed sequentially or simultaneously.

In addition, the target protein may not have to be expressed in a living body. For example, the target protein may be the component of a drug introduced from the outside into a living body. It is preferable that the drug specifically bind to a substance (such as a protein) expressed in a living body.

### (Fluorescent-immunostaining)

The fluorescent-immunostaining is a technique of staining a target protein with a fluorescence-observable dye. Here, "fluorescence" has a broad meaning, involving phosphorescence having a long luminescence lifetime in which luminescence continues even when irradiation with electromagnetic waves for excitation is stopped, and fluorescence in a narrow sense having a short luminescence lifetime.

The fluorescent-immunostaining performed by reacting a labeling antibody having a labeling substance (fluorescent substance) bound to an antibody capable of directly or indirectly binding to the target protein with a sample. The fluorescent substance is not particularly limited as long as it is an appropriate fluorescent substance for performing desired fluorescence observation, but it is preferable to use fluorophore-accumulated particles for the purpose of quantitatively detecting a target protein as a luminescent spot.

### <fluorophore-accumulated particles>

The fluorophore-accumulated particles are preferably nano-sized particles having a structure in which a plurality of fluorophores (for example, fluorescent dyes) are fixed and accumulated inside or on the surface of base particles made of an organic or inorganic substance. The fluorophore-accumulated particles used in the present invention can be prepared by a known method after selecting an appropriate raw material for forming a fluorophore and a base.

Examples of the raw material for forming the base of the particles include substances capable of stably containing a fluorescent dye such as polystyrene, polyamide, polylactic acid, polyacrylonitrile, polyglycidyl methacrylate, polymelamine, polyurea, polybenzoguanamine, polyfuran, polyxylene, phenolic resin, polysaccharide and silica. By containing the fluorophore in such particles, fluorophore-accumulated particles can be produced which are less likely to be degraded by irradiation with excitation light (higher light resistance) than the fluorophore alone. For example, hydrophobic compounds such as polystyrene, polymelamine and silica are preferable as the base of the fluorophore-accumulated particles having high light resistance.

The fluorophore contained in the fluorophore-accumulated particles is not particularly limited, but, for example, publicly known various organic fluorescent dyes or semiconductor nanoparticles (sometimes also referred to as quantum dots or the like) are preferably used.

### <Organic fluorescent dye>

Organic fluorescent dyes that can be used as a fluorophore are not particularly limited, but examples thereof may include substances known as organic fluorescent dyes such as a fluorescein-based dye, Rhodamine-based dye, Alexa Fluor (registered trademark, manufactured by Invitrogen)-based dye, BODIPY (registered trademark, manufactured by Invitrogen)-based dye, Cascade (registered trademark, manufactured by Invitrogen) - based dye, squarylium-based dye, pyrromethene-based dye, oxonol-based dye, coumarin-based dye, NBD (registered trademark)-based dye, pyrene-based dye molecule, Texas Red (registered trademark)-based dye, cyanine-based dye, perylene-based dye and oxazine-based dye. In particular, Texas Red (registered trademark)-based dye, fluorescein-based dye and pyrromethene-based dye are preferable. Specifically, Texas Red, FITC or Pyrromethene is particularly preferable.

### <Semiconductor nanoparticles>

Semiconductor nanoparticles that can be used as a fluorophore are not particularly limited, but examples thereof include semiconductor nanoparticles containing a group II-VI compound, III-V compound and IV element as a component (referred to as "II-VI semiconductor nanoparticles", "III-V semiconductor nanoparticles" and "IV semiconductor nanoparticles", respectively). Specific examples thereof include CdSe, CdS, CdTe, ZnSe, ZnS, ZnTe, InP, InN, InAs, InGaP, GaP, GaAs, Si and Ge.

Core-shell type semiconductor nanoparticles in which semiconductor nanoparticles are used as the core, and a shell is provided therearound may also be used. As a notation of semiconductor nanoparticles having a core and a shell, in the case where the core is CdSe and the shell is ZnS, when the notation is CdSe/ZnS, for example, CdSe/ZnS, CdS/ZnS, InP/ZnS, InGaP/ZnS, Si/SiO₂, Si/ZnS, Ge/GeO₂ or Ge/ZnS may be used.

### (Signal measurement)

In the step (D), a signal derived from the target protein is measured after the above steps (A) to (C). Staining in the respective steps (A) to (C) of the present invention is performed on the same sample. In other words, the signal measurement in the step (D) is performed on the same sample stained in the steps (A) to (C). The signal derived from a target protein specifically refers to a signal derived from a substance with which the target protein is labeled. The staining in the step (C) being fluorescent-immunostaining, the signal refers to a fluorescent signal derived from a fluorescent substance with which the target protein is labeled. Hereinafter, a detailed description is made of measurement of a signal derived from a target protein according to the invention where the staining in the step (A) and the step (B) is dye-immunostaining, and the staining in the step (C) is fluorescent-immunostaining.

A stained image of the fluorescent-immunostaining performed on the target protein can be obtained as a fluorescent image by irradiating a sample stained in the steps (A) to (C) with excitation light corresponding to the fluorescent substance used for the fluorescent-immunostaining in the step (C) to perform photographing in the dark field of view. The irradiation with the excitation light can be performed using a light source such as a laser light source included in a fluorescence microscope and an optical filter for excitation light that selectively transmits light with a predetermined wavelength as necessary.

The measurement of the signal derived from the target protein can be performed based on the fluorescent image of the fluorescent-immunostaining. The signal derived from the target protein is preferably the luminescent spot or the luminance of the fluorescent substance with which the target protein is labeled, more preferably the number of luminescent spots of the fluorescent substance with which the target protein is labeled. In particular, when fluorophore-accumulated particles are used as the fluorescent substance, one luminescent spot corresponds to one fluorophore-accumulated particle, so that the size is constant. Accordingly, the signal can be quantitatively measured. In this case, the luminescent spot having a size larger than a certain value (for example, the average value of the observed fluorophore-accumulated particles) per luminescent spot is judged as the aggregated luminescent spot. By dividing the luminance of the aggregated luminescent spot by the luminance per fluorophore-accumulated particle, the number of fluorophore-accumulated particles contained in the aggregated luminescent spot is calculated. The number can be regarded as the number of luminescent spots.

Furthermore, in the same field of view, photographing is performed by irradiating the sample with illumination light, whereby a stained image of the dye-immunostaining performed on the first macrophage protein and the second macrophage protein is obtained. In one embodiment of the present invention, the step (E) of identifying the position and the number of immune cells based on the dye-stained image is performed. In addition, the step (E) is preferably a step of identifying the position and the number of M2 macrophages based on the dye-immunostained image, more preferably a step of identifying the position and the number of TAMs.

Specifically, for example, when the respective macrophage proteins stained in the step (A) and the step (B) are both proteins that are specifically expressed in M2 macrophages, cells in which the respective macrophage proteins are expressed can be determined as M2 macrophages. Furthermore, for example, when one of the first macrophage protein and the second macrophage protein is a protein specifically expressed in M1 and M2 macrophages, whereas the other is a protein specifically expressed in M2 macrophages, it can be determined that cells in which the respective macrophage proteins are both expressed are M2 macrophages, whereas cells in which one macrophage protein is expressed are M1 macrophages. Furthermore, in the step (E), a region serving as a region of interest (ROI) may be set in a dye-stained image, and the position and number of macrophages contained in the region of interest may be identified. For example, TAMs can be determined by setting a tumor region contained in a sample as the region of interest.

In this way, among cells contained in a stained image, determining macrophages, preferably M2 macrophages or TAMs can identify the position and the number of them. Furthermore, from the identified position and number, for example, the number of cells of macrophages (M2 macrophages or TAMs) per unit area of a sample and the distribution thereof in the sample, and the ratio of the number of M2 macrophages or TAMs to the total number of macrophages contained in the sample can be measured.

In one embodiment of the present invention, information on the position or expression amount of the target protein in immune cells can be obtained by performing the step (F) of identifying information on the expression state of the target protein based on the signal derived from the target protein described above and the position and the number of immune cells (M2 macrophages, TAMs) identified in the step (E).

For example, information on the expression state of the target protein in macrophages can be identified by identifying the position and the number of macrophages and further extracting and measuring the signal of the target protein contained in the macrophages, based on an image in which a dye-immunostained image and a fluorescent-immunostained image are superimposed by image processing. Specifically, for example, when the target protein is a protein expressed in TAMs, it can be determined that the fluorescence in the cells determined to be TAMs in an image subjected to the image processing represents the target protein. Accordingly, this fluorescence is measured as a signal derived from the target protein.

On the other hand, for cells that were not determined to be TAMs in an image subjected to the image processing, even if fluorescence is observed in the cells, it is determined that the fluorescence is not a signal derived from the target protein expressed in TAMs, but, for example, a signal derived from the target protein expressed in tumor cells or non-specific fluorescence. In addition, in the step (F), a region serving as the region of interest (ROI) may be set in an image in which a dye-immunostained inage and a fluorescent-immunostained image are superimposed by image processing, and information on the expression state of the target protein in macrophages contained in the region of interest may be identified.

In this way, by accurately determining one of macrophages, preferably M2 macrophages, and TAMs, more preferably M2 macrophages and TAMs, and extracting and measuring the signal of the target protein contained in the macrophages, information on the expression state of the target protein can be identified in more detail. Specific examples include the average expression amount and density of the target protein per macrophage (for example, TAM) cell, the localization of the target protein in macrophages, the ratio of the expression amount of the target protein expressed in macrophages to the total expression amount of the target protein per unit area of a sample.

Examples of software that can be used for the above-described image processing, measurement of fluorescence signal and the like include image processing software "ImageJ" (open source) and full luminescent spot automatic measurement software "G-Count" (manufactured by G-Angstrom). The process of extracting the luminescent spots in a certain cell to calculate the sum of luminance, or measuring the number of luminescent spots having a predetermined luminance or higher can be semi-automatically and quickly performed using such software.

### (Immunostaining)

According to the present invention, the steps (A) to (C) are immunostaining performed on a first immune cell protein, a second immune cell protein and a target protein in the same sample, respectively, the step (C) is fluorescent-immunostaining for staining a target protein Hereinafter, a description is made of an example of the immunostaining method of the present invention.

### (Sample)

The "sample" is a tissue section collected from a subject or cells obtained by culturing a cell contained in a tissue collected from a subject. In general, such a sample is in the form of a specimen slide commonly used when evaluating the expression amount of the target protein by immunostaining, on which specimen slide the tissue section or cells are placed.

Samples to be subjected to the immunostaining method of the present invention may be derived from a normal tissue, or suitably may be derived from a lesion tissue, in particular a tumor tissue. The "tumor tissue" is a tissue containing cancer cells collected from a subject according to a standard method, and the examples may include, in addition to cancer cells, normal cells, blood vessels and stromal cells (fibroblasts, endothelial cells, leukocytes (lymphocytes, monocytes, neutrophils, eosinophils, basophils) and the like). In this case, the subject may be a human (a cancer patient or a person suspected of having cancer) or an animal other than a human. The "sample derived from a tumor tissue" is a tumor tissue section or cancer cell mass collected from a lesion of a subject having (or suspected of having) a tumor, or cells obtained by culturing tumor cells contained in a tumor tissue collected from the subject, preferably in a form of the specimen slide as described above. When the "sample derived from a tumor tissue" is a tumor tissue section, the sample may also include a surrounding normal tissue in addition to the tumor tissue. In this specification, a region containing a tumor tissue (cancer cells) in a sample derived from a tumor tissue is referred to as a "tumor region".

The method for preparing a tissue section and a specimen slide is not particularly limited. In general, for example, a specimen slide can be prepared by forming a 3 to 4 µm section of a tissue sample prepared by performing paraffin embedding in which a tissue collected from a subject is fixed using formalin or the like, subjected to dehydration treatment with alcohol and xylene treatment, and immersed in high-temperature paraffin, and placing the tissue section on a slide glass, followed by drying.

The "tumor" is not particularly limited, but is typically a solid tumor (cancer), and examples thereof include solid cancers such as cell tumor, melanoma, sarcoma, brain tumor, head and neck cancer, stomach cancer, lung cancer, breast cancer, liver cancer, colon cancer, cervical cancer, prostate cancer and bladder cancer, leukemia, lymphoma, and multiple myeloma.

The immunostaining method of the present invention is performed outside the living body of a subject using such a sample.

### (Prestaining treatment)

### (Deparaffinization treatment)

The sample is immersed in a container filled with xylene to remove paraffin. It can be performed at room temperature, although the temperature is not particularly limited. The immersion time is preferably 3 minutes or more and 30 minutes or less. If necessary, xylene may be exchanged during the immersion.

Next, the sample is immersed in a container filled with ethanol to remove xylene. It can be performed at room temperature, although the temperature is not particularly limited. The immersion time is preferably 3 minutes or more and 30 minutes or less. If necessary, ethanol may be exchanged during the immersion.

The sample is immersed in a container filled with water to remove ethanol. It can be performed at room temperature, although the temperature is not particularly limited. The immersion time is preferably 3 minutes or more and 30 minutes or less. If necessary, water may be exchanged during the immersion.

### (Activation treatment)

For the sample subjected to the deparaffinization treatment, before the step (A), preferably before the step (B) in addition to the step (A), it is preferable to perform an activation treatment of a target substance. Activation can be performed by a conventional method, and examples of the method include a method including performing heat treatment on a sample and a method including immersing a sample using a protease as an activating solution.

When a heat treatment is performed, activation can be performed using 0.01 M citrate buffer (pH 6.0), 1 mM EDTA solution (pH 8.0), 5% urea, 0.1 M Tris-HCl buffer or the like as the activating solution, and performing heating using an autoclave, microwave, pressure cooker, water bath or the like at 50 to 130°C for about 10 to 20 minutes.

When a protease is used, for example, a solution obtained by dissolving pepsin, proteinase K, trypsin or the like in PBS (phosphate buffered saline) can be used as the activating solution, and activation can be performed, for example, by immersion under a room temperature environment for 5 to 30 minutes.

Washing is performed by immersing the sample after the activation treatment in a container filled with PBS. It can be performed at room temperature, although the temperature is not particularly limited. The immersion time is preferably 3 minutes or more and 30 minutes or less, and if necessary, PBS may be exchanged during the immersion.

### (Optional treatment)

After the activation treatment, it is preferable to perform a treatment such as blocking, if necessary, for the purpose of reducing background noise and the like before performing each staining. For example, when each staining is an immunostaining, non-specific adsorption of the antibody to a sample can be suppressed by adding dropwise a surfactant such as BSA (bovine serum albumin)-containing PBS or Tween 20 as a blocking agent. In addition, for example, when an enzyme substrate reaction of peroxidase is used in dye-staining, it is preferable to perform a treatment such as a peroxidase block with hydrogen peroxide to prevent non-specific color reaction by endogenous peroxidase. Furthermore, in each step of the pre-treatment, if necessary, for example, after each treatment, it is preferable to perform an immersing treatment in a formalin solution for a certain period of time to fix a tissue slide.

After performing these treatments, it is preferable to perform washing with PBS or the like. The washing condition can be appropriately adjusted depending on the performed treatment, and the washing can be performed, for example, at room temperature for 3 minutes or more and 30 minutes or less.

### (Staining treatment)

On the sample after the washing, staining of a first macrophage protein (step (A)), staining of a second macrophage protein (step (B)), and staining for staining of a target protein (step (C)) are sequentially performed. Before each staining step, it is preferable to perform a blocking treatment in which a publicly known blocking agent such as BSA-containing PBS or a surfactant such as Tween 20 is added dropwise. Before and after each staining, it is preferable to appropriately perform washing with PBS or the like.

In immunostaining, a labeling antibody having an antibody capable of directly or indirectly binding to a protein to be detected (each of a macrophage protein and a target protein) directly or indirectly bound to a labeling substance is dispersed in an appropriate medium, placed on a sample such as a tissue section, and reacted with a target biological material to stain a protein to be detected.

A product obtained by binding a labeling substance to a primary antibody may be used as a labeling antibody, or a product obtained by binding a labeling substance to a secondary antibody may be used as a labeling antibody.

When a product obtained by binding a labeling substance to a primary antibody is used as the labeling antibody, the labeling antibody stains a macrophage protein or a target protein in a sample by directly binding to them.

When a product obtained by binding a labeling substance to a secondary antibody is used as the labeling antibody, the labeling antibody binds to the primary antibody that has been previously bound to a macrophage protein or a target protein in a sample, thereby staining the macrophage protein or the target protein in the sample.

In addition, the binding between the antibody and the labeling substance may be direct or indirect. For example, one aspect of the staining in which the labeling antibody is a secondary antibody and the secondary antibody and the labeling substance are indirectly bound includes an embodiment having [sample (target protein)]...[primary antibody (probe)]...[secondary antibody]-[biotin]/[avidin]-[labeling substance] ("..." indicates binding through antigen-antibody reaction, "-" indicates binding through covalent bond which may be via a linker molecule as needed, and "/" Indicates binding through avidin-biotin reaction). Such staining can be performed by, for example, firstly immersing a sample in a solution of the primary antibody, next immersing the sample in a solution of the secondary antibody-biotin conjugate, and finally immersing the sample in a solution of the avidin-labeling substance.

The aspect of [sample (target protein)]... [primary antibody (probe)]... [secondary antibody] - [biotin]/[avidin]-[labeling substance] may be replaced with [sample (target protein)]... [primary antibody (probe)]...[secondary antibody]-[hapten]/[anti-hapten antibody]-[labeling substance], and the hapten may include DIG, DNP, FITC and Cy3.

### [Primary antibody]

The primary antibody is an antibody capable of specifically binding to a protein to be stained by recognizing the unique epitope, and may be a polyclonal antibody, but is preferably a monoclonal antibody from the viewpoint of stability of quantification. For example, when HER2 is selected as a target protein for staining, an anti-HER2 antibody can be used. As the primary antibody, an antibody of any isotype may be used as long as it can specifically recognize a target substance, but an IgG antibody (immunoglobulin G) is particularly preferably used. The primary antibody does not need to have full-length, unlike a natural antibody, as long as it can bind to a target substance, and may be an antibody fragment or derivative, a chimeric antibody (such as a humanized antibody), or a multifunctional antibody.

### [Secondary antibody]

The secondary antibody is an antibody that does not bind to a protein to be stained itself contained in a sample, but specifically recognizes and binds to a part of a primary antibody bound to the protein to be stained which part has not reacted with a target substance (for example, Fc, F(ab) or F (ab')). As the secondary antibody, an anti-IgG antibody can be suitably used. When the primary antibody is hapten-modified, an anti-hapten antibody capable of recognizing hapten can also be used for achieving the same effect.

The type of animal for producing the primary antibody and the secondary antibody (immunized animal) is not particularly limited, but may be selected from mice, rats, guinea pigs, rabbits, goats and sheep, similarly to the related art. Usually, the secondary antibody is produced in a different type of immunized animal from that selected for the primary antibody.

Conditions for performing immunostaining, for example, the temperature and treating time for performing immunostaining can be adjusted appropriately to produce an appropriate signal
according to a conventional immunostaining method, and for example, the reaction can be performed at room temperature for 30 minutes or more and 24 hours or less.

### (Optional staining)

In addition to the above staining, a substance other than the macrophage protein and the target protein possessed by cells (for example, nuclear) may be stained through binding with a fluorescent substance (for example, nuclear staining agent: Nuclear Fast Red), or stained with a staining agent for morphological observation such that the shape of cells can be identified (for example, Eosin).

The optional staining may be performed according to a conventional method. The optional staining is preferably performed after the staining of the macrophage protein and the target protein to be stained, that is, after the step (C).

### (Post-staining treatment)

After the staining step, the specimen slide is preferably subjected to a treatment such as fixation/dehydration, clearing and encapsulation so as to be suitable for photographing for signal measurement and the like as described above.

The fixation/dehydration treatment may be performed by immersing the specimen slide in a solution for fixation treatment (crosslinking agent such as formalin, paraformaldehyde, glutaraldehyde, acetone, ethanol and methanol). The clearing may be performed by immersing the specimen slide subjected to the fixation/dehydration treatment in a clearing solution (xylene or the like). The encapsulation treatment may be performed by immersing the specimen slide subjected to the clearing treatment in an encapsulating solution. Conditions for performing these treatments, for example, the temperature and immersion time for immersing the specimen slide in the predetermined solution for treatment can be appropriately adjusted according to a conventional immunostaining method so as to produce an appropriate signal.

### Examples

Hereinafter, more specific description is made of preferred aspects of the present invention with reference to Examples, but the present invention is not limited to these Examples.

### [Preparation Example 1]

### (Preparation of biotin-modified anti-rabbit IgG antibody)

In a 50 mM Tris solution, 50 µg of an anti-rabbit IgG antibody (LO-RG-1 from AbD) was dissolved. To this solution, a DTT (dithiothreitol) solution was added to a final concentration of 3 mM, followed by mixing to react at 37°C for 30 minutes. Thereafter, the reaction solution was passed through a desalting column "Zeba Desalt Spin Columns" (from Thermo Scientific, Cat. # 89882) to purify a secondary antibody reduced with DTT. In a 50 mM Tris solution, 200 µL of the total amount of the purified antibody was dissolved to prepare an antibody solution. Meanwhile, a linker reagent "Maleimide-PEG2-Biotin" (from Thermo Scientific, product number 21901) was adjusted to 0.4 mM using DMSO. To the antibody solution, 8.5 µL of the linker reagent solution was added, followed by mixing to react at 37°C for 30 minutes, thereby binding biotin to the anti-rabbit IgG antibody via a PEG chain. This reaction solution was passed through a desalting column for purification. The absorbance at a wavelength of 300 nm for the desalted reaction solution was measured using a spectrophotometer ("F-7000" manufactured by Hitachi, Ltd.) to calculate the concentration of the protein (biotin-modified IgG antibody) in the reaction solution, and the concentration of the biotin-modified IgG antibody was adjusted to 250 µg/mL using a 50 mM Tris solution.

### [Preparation Example 2]

### (Preparation of FITC-modified anti-mouse IgG2b antibody)

Using an FITC labeling kit (Fluorescein Labeling kit-NH2/LK01: manufactured by Dojin Molecular Technologies, Inc.) according to the instruction for the kit, an FITC reagent (NANOCS product number: PG2-FCNS-2k) was reacted with an anti-mouse IgG2b antibody (from SouthernBiotech; clone SB74g) to prepare an FITC-labeled anti-mouse IgG antibody.

### [Preparation Example 3]

### (Preparation of FITC-modified anti-mouse IgG2a antibody)

Using a labeling kit (biotin Labeling kit-SH/LK10: manufactured by Dojin Molecular Technologies, Inc.) according to the instruction for the kit, firstly, an anti-mouse IgG2a antibody (from Southern Biotech (SBA); clone: SB84a) was reduced. Next, an FITC-labeled anti-mouse IgG2a antibody was prepared by reacting with an FITC reagent (FITC-5-maleimide (from Tokyo Chemical Industry Co., Ltd., F0810)) instead of a biotin reagent included in the kit.

### [Preparation Example 4]

### (Preparation of streptavidinated Texas Red-accumulated particles)

After dissolving 2.5 mg of Texas Red dye molecule "Sulforhodamine 101" (from Sigma-Aldrich) in 22.5 mL of pure water, the solution was stirred for 20 minutes with a hot stirrer while maintaining the temperature at 70°C. To the solution subjected to the stirring, 1.5 g of a melamine resin "Nikalac MX-03 5" (from Nippon Carbide Industries Co., Inc.) was added, followed by further heating and stirring under the same condition for 5 minutes.

To the solution subjected to the stirring, 100 µL of formic acid was added. Subsequently, the solution was stirred for 20 minutes while maintaining the temperature at 60°C, and then left to be cooled to room temperature. The cooled solution was dispensed into a plurality of centrifugation tubes, and centrifuged at 12,000 rpm for 20 minutes to precipitate melamine resin particles including the Texas Red dye contained as a mixture in the solution. The supernatant was removed and the precipitated particles were washed with ethanol and water. SEM observation was performed on 1,000 obtained resin particles, and the average particle diameter was measured as described above. As a result, the average particle diameter was 152 nm. The prepared melamine resin particles containing the Texas Red dye in this way were surface-modified by the following procedure.

In 1.5 mL of EtOH, 0.1 mg of the obtained particles were dispersed and 2 µL of amine propyltrimethoxysilane LS-3150 (manufactured by Shin-Etsu Chemical Co., Ltd.) was added to react for 8 hours for surface amination treatment.

Next, the particles subjected to the surface amination treatment were adjusted to 3 nM using PBS (phosphate buffered saline) containing 2 mM EDTA (ethylenediaminetetraacetic acid). Then, with this solution, SM (PEG)₁₂ (manufactured by Thermo Scientific, succinimidyl-[(N-maleimidopropionamido)-dodecaethyleneglycol] ester) was mixed to a final concentration of 10 mM to react for 1 hour. The mixture was centrifuged at 10,000 G for 20 minutes. After the supernatant was removed, PBS containing 2 mM EDTA was added to disperse the precipitate, followed by centrifugation again. The same procedure for washing was repeated three times to produce maleimide-modified Texas Red-accumulated melamine particles.

On the other hand, streptavidin (manufactured by Wako Pure Chemical Corporation) was subjected to a thiol group addition treatment using N-succinimidyl S-acetylthioacetate (SATA), followed by filtration through a gel filtration column to prepare thiol-modified streptavidin.

The maleimide-modified Texas Red-accumulated melamine particles and thiol-modified streptavidin were mixed in PBS containing 2 mM EDTA to react at room temperature for 1 hour. The reaction was stopped by adding 10 mM mercaptoethanol. After the obtained solution was concentrated with a centrifugal filter, unreacted streptavidin and the like were removed using a gel filtration column for purification, and the obtained streptavidin-bound Texas Red-accumulated melamine particles were recovered. The obtained streptavidin-bound Texas Red-accumulated melamine particles were dispersed in PBS containing 1% BSA and diluted to 0.09 au.

### [Preparation Example 5]

### (Preparation of anti-FITC antibody-bound Pyrromethene 556-accumulated melamine resin particles)

To 22 mL of water, 14.4 mg of Pyrromethene 556 as a green fluorescent dye was added, followed by dissolution. Thereafter, to this solution, 2 mL of a 5% aqueous solution of "Emulgen" (registered trademark) 430 (polyoxyethylene oleyl ether, manufactured by Kao Corporation), an emulsifier for emulsion polymerization was added. After the temperature of the solution was raised to 70°C while being stirred on a hot stirrer, to the solution was added 0.35 g of melamine resin raw material "Nikalac MX-035" (manufactured by Nippon Carbide Industries Co., Inc.).

To the solution was added 1000 µL of a 10% aqueous solution of dodecylbenzenesulfonic acid (manufactured by Kanto Chemical Co., Ltd.) as a reaction initiator, followed by heating and stirring at 70°C for 50 minutes. Thereafter, the temperature was raised to 90°C, followed by further heating and stirring for 20 minutes.

Washing with pure water was performed to remove excess resin raw material and impurities such as the fluorescent dye from the obtained dispersion of pyrromethene-accumulated melamine resin particles. Specifically, a procedure was repeated five times in which centrifugation at 20000 G for 15 minutes in a centrifuge "micro cooling centrifuge 3740" (manufactured by Kubota Corporation) was performed for removing the supernatant, then ultrapure water was added, and re-dispersion was achieved by ultrasonic irradiation.

According to the above steps, pyrromethene-accumulated melamine resin particles (excitation wavelength of 490 nm, emission wavelength of 520 nm) were prepared. The average particle diameter was 79 nm.

To the end of the pyrromethene-accumulated melamine resin particles, maleimide was introduced using an NHS-PEG (polyethylene glycol)-maleimide reagent (SM (PEG)₁₂ (manufactured by Thermo Scientific, succinimidyl-[(N-maleimidopropionamido)-dodecaethyleneglycol]ester), and then to the maleimide, a thiolated anti-FITC antibody (manufactured by abcam; [2A3] (ab 10257)) was bound in the same procedure as in the thiolation of streptavidin in the Preparation Example 1.

### [Preparation Example 6]

### (Preparation of anti-FITC antibody-modified Pyrromethene 556-accumulated silica particles)

To 13.4g of pyrromethene 556 was added 0.1 mL of thionyl chloride, followed by heating and mixing for 654 hours, and then vacuum drying was performed to remove excess thionyl chloride. The obtained reaction product of pyrromethene 556 and thionyl chloride, and 3 µL of 3-aminopropyltrimethoxysilane (3-aminopropyltrimetoxysilane, manufactured by Shin-Etsu Chemical Co., Ltd., KBM903) were mixed in 1.2 mL of N,N-dimethylformamide (DMF) to prepare an organoalkoxysilane compound.

With 24 mL of 99% ethanol, 0.3 mL of tetraethoxysilane (TEOS), 0.75 mL of ultrapure water and 0.75 mL of 28% by mass ammonia water was mixed 0.3 mL of a solution of the obtained organoalkoxysilane compound at 25°C for 1 hour.

The mixture prepared in the above step was centrifuged at 10,000 G for 20 minutes to remove the supernatant. Ethanol was added to the precipitate to disperse the precipitate for rinsing, followed by centrifugation again. Furthermore, the same rinsing was repeated twice to prepare pyrromethene 556-accumulated silica particles. The average particle diameter was 122 nm.

On the pyrromethene 556-accumulated silica particles, maleimide modification was performed in the same manner as in the Preparation Example 4, and to the maleimide-modified pyrromethene 556-accumulated silica particles, an anti-FITC antibody (manufactured by abcam; [F4/1] (ab 112511)) instead of the anti-FITC antibody (manufactured by abcam; [2A3] (ab10257)) was bound in the same manner as in the Preparation Example 5 to prepare anti-FITC antibody-modified pyrromethene 556-accumulated silica particles.

### [Example 1]

### (1) Prestaining treatment

### (1-1) Deparaffinization treatment

A lung adenocarcinoma tissue array slide (HLug-Ade150Sur-02: from US Biomax) was deparaffinized according to the following procedure. The paraffin in the slide was melted by placing the tissue array slide in a 65°C incubator for 15 minutes. The slide was immersed in three containers filled with xylene for 5 minutes each, washed with dehydrated ethanol (Kanto Chemical Co., Ltd.; 14599-95), and further immersed in dehydrated ethanol twice for 5 minutes each. Thereafter, dehydration was further performed with 99.5% ethanol (Kanto Chemical Co., Ltd.; 14033-70), and washing was performed in running pure water for 10 minutes.

### (1-2) Activation treatment

The deparaffinized tissue array slide is immersed in an activating solution (10 mM Tris buffer (pH 9.0)) preheated to 95°C, and left for 40 minutes. After being left at room temperature, the slide is washed with running pure water for 10 minutes, and further the section slide is immersed in a staining tray filled with PBS and washed 3 times for 5 minutes each.

### (1-3) Endogenous peroxidase block

The activated tissue array slide was immersed in 3% hydrogen peroxide for 15 minutes to perform endogenous peroxidase block.

### (1-4) Blocking

The treated tissue array slide was immersed in PBS containing 1% BSA for blocking treatment at room temperature for 15 minutes.

### (2) CD68 staining step

### (2-1) Primary antibody reaction

Anti-CD68 mouse monoclonal antibody [PG-M1] (from Dako) was diluted 100 times using PBS containing 1% BSA, and then added to the tissue array slide subjected to the blocking treatment, followed by reaction at room temperature for 1 hour.

### (2-2) Secondary antibody reaction

After the tissue array slide subjected to the primary antibody reaction was washed with PBS, Histofine Simple Stain MAX-PO (MULTI) (from Nichirei Biosciences Inc.; 049-22831) was added, followed by reaction at room temperature for 30 minutes.

### (2-3) DAB staining

After the tissue array slide subjected to the secondary antibody reaction was washed with PBS, DAB adjusted with 0.05 mol/L Tris-HCl buffer (pH 7.6) and charged with 30% hydrogen peroxide just before use was added, followed by reaction at room temperature for 3 minutes and then immersion in pure water for 5 minutes.

### (3) CD163 staining step

### (3-1) Activation treatment

The tissue array slide subjected to the DAB staining was reactivated in the same manner as in (1-2).

### (3-2) Blocking

The tissue array slide subjected to the activation treatment in (3-1) was blocked in the same manner as in (1-4).

### (3-3) Primary antibody reaction

An anti-CD163 mouse monoclonal antibody (manufactured by abcam; [10D6]) is diluted 50 times using PBS containing 1% BSA, and then added to the tissue array slide subjected to the blocking treatment in (3-2), followed by reaction at 4°C overnight.

### (3-4) Secondary antibody reaction

The tissue array slide subjected to the blocking treatment in (3-3) was subjected to a secondary antibody reaction in the same manner as in (2-2).

### (3-5) HistoGreen staining

After the tissue array slide subjected to the secondary antibody reaction (3-4) is washed with PBS, HistoGreen (from AbCys; E109) is added, followed by reaction at room temperature for 3 minutes. After the reaction, the slide was washed with PBS three times for 5 minutes each, and further washed with pure water.

### (4) MCSFR (CSF-1R) fluorescent-staining step

### (4-1) Blocking

The tissue array slide subjected to the washing in (3-4) was blocked in the same manner as in (1-4).

### (4-2) Primary antibody reaction

An anti-MCSFR rabbit monoclonal antibody (manufactured by abcam; [SP211]) was diluted 50 times using PBS containing 1% BSA, and then added to the blocked tissue array slide, followed by reaction at 4°C overnight.

### (4-3) Secondary antibody reaction

After the tissue array slide subjected to the primary antibody reaction was washed with PBS, the biotinylated secondary antibody prepared in the Preparation Example 1 diluted to 2 µg/mL with PBS containing 1% BSA was added, followed by reaction for 30 minutes.

### (4-4) Fluorescent labeling

After the tissue array slide subjected to the secondary antibody reaction was washed with PBS, the dispersion of the streptavidinated Texas Red-accumulated particles prepared in the Preparation Example 4 was added, followed by reaction at room temperature for 2 hours. After the 2 hours, washing was performed with PBS 3 times for 5 minutes each, and 4% PFA was added to the section slide, followed by reaction for 10 minutes.

### (5) Cell nuclear staining step

The tissue array slide subjected to the PFA reaction was exposed to running pure water for 1 minute, and then immersed in a Mayer's hematoxylin solution for 1 minute.

### (6) Encapsulation step

### (6-1) Dehydration/clearing

The section slide subjected to the hematoxylin staining was transferred to a washing tank, and exposed to running pure water for 10 minutes. Thereafter, the section slide was immersed in "99.5% EtOH tank", "dehydrated EtOH tank" × 3 and "xylene tank" × 3 in this order.

### (6-2) Encapsulation

The section slide subjected to the dehydration/clearing was encapsulated by an automatic encapsulation machine. The section slide was then stored under light protection.

### (7) Photographing step

Firstly, using a digital camera for microscope "DP73" (Olympus Corporation) attached to a fluorescence microscope "BX-53" (Olympus Corporation), a dye-stained image (400 ×) was photographed.

Next, a fluorescent image was photographed using a fluorescent microscope "BX-53" (Olympus Corporation). The specimen was irradiated with excitation light corresponding to the biotinylated fluorophore-accumulated particles used in the fluorescence labeling in (4-4) to generate fluorescence, and the stained image in that state was photographed. In this case, the wavelength of the excitation light was set to 575 to 600 nm using an optical filter for excitation light provided in the fluorescence microscope, and the wavelength of the fluorescence to be observed was set to 612 to 692 nm using an optical filter for fluorescence. The intensity of the excitation light at the time of observation with the fluorescence microscope and image photographing was such that the irradiation energy near the center of the field of view was 900 W/cm². The exposure time at the time of image photographing was adjusted within a range where the luminance of the image was not saturated, for example, set to 4000 µsec.

The dye-stained image and fluorescent image were overlaid on each other using image processing software "ImageJ" (open source) to perform image processing. Among 150 samples contained in the tissue array slide, cells stained with both DAB and HistGreen, in other words, cells in which CD68 and CD 163 were stained were identified as TAMs from the result of the overlaid images, and a sample in which TAMs were present was extracted. For the sample in which TAMs were present, the luminescent spot derived from CSF-1R per TAM cell was further measured. Note that the number of luminescent spots representing the fluorophore-accumulated particles whose luminance was equal to or higher than a predetermined value was measured. In addition, since macrophages present in a tumor tissue are mainly M2 macrophages, in the Examples, the number of M2 macrophages was defined as the number of TAMs. Alternatively, the number (proportion) of M1 macrophages and the number (proportion) of M2 macrophages in TAMs may be individually determined.

Table 1 shows the number of TAMs contained in the image and the average value of the number of luminescent spots per TAM cell for the sample in which TAMs are present. It can be seen that the number of contained TAMs and the number of luminescent spots per TAM cell (the expression amount of CSF-1R) vary for each sample.

**[Table 1]**

| SAMPLE No. | AVERAGE NUMBER OF TAMS | AVERAGE NUMBER OF LUMINESCENT SPOTS (SPOTS/CELL) |
|---|---|---|
| F3 | 8 | 77 |
| D3 | 8 | 111 |
| B9 | 19 | 148 |
| A15 | 6 | 163 |
| B11 | 15 | 175 |
| E9 | 11 | 194 |
| E3 | 12 | 198 |
| B3 | 12 | 201 |
| B7 | 9 | 222 |
| A1 | 14 | 240 |
| A5 | 5 | 241 |
| 19 | 10 | 262 |
| G3 | 8 | 303 |
| J5 | 21 | 318 |
| F7 | 4 | 323 |
| C9 | 18 | 360 |
| C15 | 20 | 371 |
| 15 | 7 | 436 |
| F9 | 9 | 451 |
| B13 | 4 | 515 |
| E11 | 4 | 534 |
| H13 | 14 | 581 |
| A13 | 11 | 642 |
| B15 | 3 | 665 |

### [Example 2]

In the same manner as in Example 1, (1) a prestaining step, (2) a CD68 staining step, and (3) a CD163 staining step were performed.

In the (4) fluorescent-staining step of Example 1, subsequent to CSF-1R staining, IDO staining was performed. Hereinafter, a detailed description is made of the fluorescent-staining step as a fluorescent-staining step (4').

### (4') Fluorescent-staining step

### (4'-1) Blocking

In the same manner as in Example 1, after the (2) CD68 staining step and (3) CD163 staining step were performed, and CSF-1R fluorescent-staining was performed, the washed tissue array slide was blocked in the same manner as in (1-4).

### IDO fluorescent-staining step

### (4'-2) Primary antibody reaction

A solution in which an anti-IDO mouse monoclonal antibody (manufactured by abcam/Mouse IgG2b/ab55305) was diluted 100 times using PBS containing 1% BSA was added to the blocked tissue array slide, followed by reaction at 4°C overnight.

### (4'-3) Secondary antibody reaction

After the tissue array slide subjected to the primary antibody reaction was washed with PBS, the FITC-modified anti-mouse IgG2b antibody prepared in the Preparation Example 2 diluted to 2 µg/mL with PBS containing 1% BSA was added, followed by reaction for 30 minutes.

### (4'-4) Fluorescent labeling

After the tissue array slide subjected to the secondary antibody reaction was washed with PBS, the dispersion of the anti-FITC antibody-bound pyrromethene-accumulated melamine resin particles prepared in the Preparation Example 5 was added, followed by reaction at room temperature for 2 hours. After the 2 hours, washing was performed with PBS 3 times for 5 minutes each, and 4% PFA was added to the section slide, followed by reaction for 10 minutes.

In the same manner as in Example 1, the (5) cell nuclear staining step, the (6) encapsulation treatment step, and the (7) photographing step were performed on the tissue array slide subjected to the fluorescent labeling.

(7) Dye-stained images (400 times) in the photographing step (CD68 staining and CD163 staining) were photographed in the same manner as in Example 1.

The wavelength of the excitation light when Texas Red, that is, CSF-1R was observed and photographed was set to 575 to 600 nm as in Example 1, and the wavelength of the fluorescence to be observed was set to 612 to 692 nm. In addition, the wavelength of the excitation light when pyrromethene, that is, IDO was observed and photographed was set to 420 to 520 nm, and the wavelength of the fluorescence to be observed was set to 517.5 to 532.5 nm.

### (Discussion)

As described above, in the Examples, the first macrophage protein and the second macrophage protein (CD68 and CD163), and a plurality of target proteins (CSF-1R and IDO) are observed. CSF-1R is a protein expected as a target protein for cancer treatment, and its inhibition has been shown to produce an effect of reducing TAMs, thereby suppressing tumor growth. IDO is known to be involved in checkpoint inhibition of cancer cells. From the viewpoint that tumor growth can be suppressed by inhibiting this mechanism, it is known that IDO is also a target protein for cancer treatment, and blocking CSF-1R signal transduction by IDO inhibitor induces tumor regression (EBioMedicine. 2016 Apr; 6: 50-58.).

As in the Examples, for example, it can be expected that the prognosis of patient from a sample can be predicted in clinical practice by identifying TAMs based on the first macrophage protein and the second macrophage protein, and further simultaneously detecting CSF-1R and IDO in the macrophages. For example, for a sample in which TAMs are present, when the number of TAMs contained in the image and the average number of luminescent spots for each TAM cell were determined, it can be expected that the higher number of luminescent spots results in worse prognosis of the patient.

This is because many luminescent spots indicating many target proteins, that is, target proteins for cancer treatment, CSF-1R and IDO in this case mean that tumor has progressed beyond a certain level (in other words, the microenvironment in a tumor tissue is in a state of enhanced tumor growth). Therefore, it can be predicted that the less the luminescent spots (the less the target protein, that is, the relatively lower the stage), the better the prognosis, and also the smaller the number of cells expressing the target protein, the less the remainder surviving a drug. Conversely, it can be predicted that, when the number of cells expressing the target protein is higher, even in a case where the tumor-enhancing functions of most target proteins are inactivated by a drug so that the tumor temporarily appears to have remitted, cells in which the target protein is not inactivated will survive and grow again, with the result that there is high possibility that the tumor will recur.

In this way, performing the information acquisition system of the present invention using two types of proteins, particularly CSF-1R and a protein that functions for tumor enhancement in relation to CSF-1R, as target proteins can provide useful information on treatment policy, such as predictions about the prognosis of patient, and the like.

### [Example 3]

In the same manner as in Example 1, (1) a prestaining step, (2) a CD68 staining step, and (3) a CD163 staining step were performed.

In the (4) fluorescent-staining step of Example 1, subsequent to CSF-1R staining, CXCR2 staining was performed. Hereinafter, a detailed description is made of the fluorescent-staining step as a fluorescent-staining step (4").

### (4") Fluorescent-staining step

### (4"-1) Blocking

After the (2) CD68 staining step and (3) CD163 staining step were performed, and CSF-1R fluorescent-staining was performed, the washed tissue array slide was blocked in the same manner as in (1-4).

### (4"-2) Primary antibody reaction

A solution in which an FITC-modified anti-CXCR2 mouse monoclonal antibody (manufactured by R&D SYSTEMS, Clone: 48311) was diluted 50 times using PBS containing 1% BSA was added to the blocked tissue array slide, followed by reaction at 4°C overnight.

### (4"-3) Secondary antibody reaction

After the tissue array slide subjected to the primary antibody reaction was washed with PBS, the FITC-modified anti-mouse IgG2a antibody prepared in the Preparation Example 3 diluted to 2 µg/mL with PBS containing 1% BSA was added, followed by reaction for 30 minutes.

### (4"-4) Fluorescent labeling

After the tissue array slide subjected to the secondary antibody reaction was washed with PBS, the anti-FITC antibody-labeled anti-FITC antibody-bound pyrromethene-accumulated melamine resin particles prepared in the Preparation Example 6 was added, followed by reaction at room temperature for 2 hours. After the 2 hours, washing was performed with PBS 3 times for 5 minutes each, and 4% PFA was added to the section slide, followed by reaction for 10 minutes.

In the same manner as in Example 1, the (5) cell nuclear staining step and the (6) encapsulation treatment step were performed on the tissue array slide subjected to the fluorescent labeling.

(7) Dye-stained images (400 times) in the photographing step (CD68 staining and CD163 staining) were photographed in the same manner as in Example 1.

The wavelength of the excitation light when Texas Red, that is, CSF-1R was observed and photographed was set to 575 to 600 nm as in Example 1, and the wavelength of the fluorescence to be observed was set to 612 to 692 nm. In addition, the wavelength of the excitation light when pyrromethene 556, that is, CXCR2 was observed and photographed was set to 420 to 520 nm, and the wavelength of the fluorescence to be observed was set to 517.5 to 532.5 nm.

The dye-stained image and fluorescent image were overlaid on each other using image processing software "ImageJ" (open source) to perform image processing. Cells stained with both DAB and HistGreen, that is, cells in which CD68 and CD 163 are stained are judged as TAMs, whereby a sample in which TAMs are present can be extracted.

For the sample in which TAMs were present, the luminescent spot derived from CSF-1R per TAM cell was further measured. Note that the number of luminescent spots representing the fluorophore-accumulated particles whose luminance was equal to or higher than a predetermined value was measured. In addition, since macrophages present in a tumor tissue are mainly M2 macrophages, in the Examples, the number of M2 macrophages was defined as the number of TAMs. Alternatively, the number (proportion) of M1 macrophages and the number (proportion) of M2 macrophages in a sample may be individually determined.

### (Discussion)

Similarly to the IDO used in Example 2, it is known that CXCR2 used as one of the target proteins in the Examples (its ligand CXCL1 enhances tumor growth by participating in angiogenesis in the tumor microenvironment) is also a protein whose inhibition together with CSF-1R significantly reduces tumor growth. Accordingly, performing the information acquisition system of the present invention by similarly analyzing the number of luminescent spots of the two proteins can provide useful information on treatment policy, such as predictions about the prognosis of patient, and the like.

### [Example 4]

The information acquisition system of the present invention was performed in the same manner as in Example 2, except that in the fluorescent-staining step of Example 2, an anti-PD-Ll antibody (manufactured by Medical & Biological Laboratories Co., Ltd., clone: 27A2) was used instead of the anti-IDO antibody.

### (Discussion)

PD-L1 used as one of the target proteins in Example 4 is a protein expressed on the surface of an antigen presenting cell. It is known that PD-L1 is a protein whose inhibition alone can inhibit tumor growth, but similarly to proteins such as IDO used in the above Examples, inhibition together with CSF-1R significantly reduces tumor growth (Japanese Journal of Clinical Immunology (Vol. 40 No. 4)). Also, for these, similarly to the above Examples, performing the information acquisition system of the present invention by analyzing the number of luminescent spots of CSF-1R/PD-L1 can provide useful information on treatment policy, such as predictions about the prognosis of patient, and the like.

### [Example 5]

In the staining step of Example 1, in the (2) CD68 staining step, killer T cells were stained by changing the anti-CD68 mouse monoclonal antibody to an anti-CD8 antibody (manufactured by Dako, model number: M7103, Clone: c8 / 144B), and in the (3) CD163 staining step, regulatory T cells were stained by changing the anti-CD 163 mouse monoclonal antibody to an anti-FoxP3 antibody (manufactured by Epitomics, model number: AC-0304RUO, Clone: EP340).

In the same manner as in Example 1, the (5) cell nuclear staining step and the (6) encapsulation treatment step were performed, and further the image obtained by similarly performing the (7) photographing step was analyzed to identify cells and measure luminescent spots.

Here, when PD-L1 was stained, an FITC-modified anti-PD-Ll antibody was prepared by FITC-modifying an anti-PD-Ll antibody (manufactured by Medical & Biological Laboratories Co., Ltd., clone: 27A2/cord. D230-3) in the same manner as in Preparation Example 2. In (4-2) of Example 1, the FITC-modified anti-PD-Ll antibody was reacted instead of the anti-MCSFR rabbit monoclonal antibody, and then in (4-4) fluorescent labeling of Example 1, the anti-FITC antibody-modified pyrromethene 556-accumulated silica particles prepared in Preparation Example 6 were reacted instead of the streptavidinated Texas Red-accumulated particles.

### (Discussion)

From the result of the CD8-stained image, the stained cells could be distinguished from killer T cells, and from the result of the FoxP3 -stained image, the stained cells could be distinguished from regulatory T cells. In addition, from the positional relationship between these cells and the PD-L1 luminescent spots, a distribution map showing how PD-L1 is distributed in each cell could be created. This distribution map is expected to vary for each patient, and is considered useful information.

## Claims

1. An immunostaining method comprising the following step (A) to (D):
a step (A) of staining a first immune cell protein expressed in one or more cells selected from macrophages, T cells, natural killer cells, dendritic cells, B cells, granulocytes and plasma cells;
a step (B) of staining a second immune cell protein expressed in one or more cells selected from macrophages, T cells, natural killer cells, dendritic cells, B cells, granulocytes and plasma cells;
a step (C) of staining a target protein; and
a step (D) of, after the steps (A) to (C), measuring a signal derived from the target protein,
wherein
the steps (A) to (C) are performed on a same sample, the sample being a tissue section collected from a subject or cells obtained by culturing a cell contained in a tissue collected from a subject, and the target protein being at least one protein expressed in the sample, and wherein
the staining in the steps (A) and (B) is dye-immunostaining
the staining in the step (C) is fluorescent-immunostaining and
the fluorescent-immunostaining in the step (C) is performed after the steps (A) and (B).

2. The immunostaining method according to claim 1, further comprising a step (E) of identifying a position and number of an immune cell based on the staining in the steps (A) and (B).

3. immunostaining method according to claim 1 or 2, further comprising a step (F) of identifying information on an expression state of the target protein based on the signal derived from the target protein measured in the step (D).

4. The immunostaining method according to claim 2 or 3, further comprising a step (F) of identifying information on an expression state of the target protein based on the position and number of the immune cell identified in the step (E).

5. The immunostaining method according to any one of claims 1 to 4, wherein the first immune cell protein in the step (A) and the second immune cell protein in the step (B) are individually stained with different dyes.

6. The immunostaining method according to any one of claims 1 to 5, wherein the first immune cell protein in the step (A) is stained with 3,3'-diaminobenzidine, and the second immune cell protein in the step (B) is stained with Histogreen, and
the step (B) is performed after the step (A).

7. The immunostaining method according to any one of claims 1 to 6, wherein at least one of the first immune cell protein and the second immune cell protein comprises a protein having at least one or more effects selected from a T cell response-enhancing effect and an antitumor effect.

8. The immunostaining method according to any one of claims 1 to 6, wherein at least one of the first immune cell protein and the second immune cell protein is a protein expressed in a macrophage.

9. The immunostaining method according to claim 8, wherein the first immune cell protein is a first macrophage protein that is expressed in a macrophage, and the second immune cell protein is a second macrophage protein that is expressed in a macrophage and is different from the first macrophage protein.

10. The immunostaining method according to any one of claims 1 to 9, wherein the target protein is a protein expressed in a macrophage.

11. The immunostaining method according to any one of claims 1 to 10, , wherein the signal derived from the target protein is measured as a number of luminescent spots derived from the fluorescent-immunostained target protein.

## Patentansprüche

1. Immunfärbeverfahren, das die folgenden Schritte (A) bis (D) umfasst:
einen Schritt (A) des Färbens eines ersten Immunzellproteins, das in einer oder mehreren Zellen exprimiert wird, die aus Makrophagen, T-Zellen, natürlichen Killerzellen, dendritischen Zellen, B-Zellen, Granulozyten und Plasmazellen ausgewählt sind;
einen Schritt (B) des Färbens eines zweiten Immunzellproteins, das in einer oder mehreren Zellen exprimiert wird, die aus Makrophagen, T-Zellen, natürlichen Killerzellen, dendritischen Zellen, B-Zellen, Granulozyten und Plasmazellen ausgewählt sind;
einen Schritt (C) des Färbens eines Zielproteins; und
einen Schritt (D), in dem nach den Schritten (A) bis (C) ein von dem Zielprotein abgeleitetes Signal gemessen wird,
wobei
die Schritte (A) bis (C) an derselben Probe durchgeführt werden, wobei es sich bei der Probe um einen von einem Patienten entnommenen Gewebeschnitt oder um Zellen handelt, die durch Kultivierung einer in einem von einem Patienten entnommenen Gewebe enthaltenen Zelle erhalten wurden, und das Zielprotein mindestens ein in der Probe exprimiertes Protein ist, und wobei
die Färbung in den Schritten (A) und (B) eine Immunfärbung mit einem Färbemittel ist,
die Färbung in Schritt (C) eine Immunfärbung mittels Immunfluoreszenzfärbung ist, und
die Immunfärbung mittels Immunfluoreszenzfärbung im Schritt (C) nach den Schritten (A) und (B) durchgeführt wird.

2. Immunfärbeverfahren nach Anspruch 1, ferner umfassend einen Schritt (E) der Identifizierung einer Position und Anzahl einer Immunzelle auf der Grundlage der Färbung in den Schritten (A) und (B).

3. Immunfärbeverfahren nach Anspruch 1 oder 2, ferner umfassend einen Schritt (F) zur Identifizierung von Informationen über einen Expressionszustand des Zielproteins auf der Grundlage des in Schritt (D) gemessenen, von dem Zielprotein abgeleiteten Signals.

4. Immunfärbeverfahren nach Anspruch 2 oder 3, ferner umfassend einen Schritt (F) der Identifizierung von Informationen über einen Expressionszustand des Zielproteins auf der Grundlage der in Schritt (E) identifizierten Position und Anzahl der Immunzelle.

5. Immunfärbeverfahren nach einem der Ansprüche 1 bis 4, wobei das erste Immunzellprotein in Schritt (A) und das zweite Immunzellprotein in Schritt (B) individuell mit verschiedenen Färbemitteln gefärbt werden.

6. Immunfärbeverfahren nach einem der Ansprüche 1 bis 5, wobei das erste Immunzellprotein in Schritt (A) mit 3,3'-Diaminobenzidin gefärbt wird und das zweite Immunzellprotein in Schritt (B) mit Histogreen gefärbt wird, und
der Schritt (B) nach dem Schritt (A) durchgeführt wird.

7. Immunfärbeverfahren nach einem der Ansprüche 1 bis 6, wobei mindestens eines von dem ersten Immunzellprotein und dem zweiten Immunzellprotein ein Protein umfasst, das mindestens eine oder mehrere Wirkungen hat, die aus einer die T-Zell-Antwort-stimulierenden Wirkung und einer Antitumorwirkung ausgewählt sind.

8. Immunfärbeverfahren nach einem der Ansprüche 1 bis 6, wobei mindestens eines von dem ersten Immunzellprotein und dem zweiten Immunzellprotein ein in einem Makrophagen exprimiertes Protein ist.

9. Immunfärbeverfahren nach Anspruch 8, wobei das erste Immunzellprotein ein erstes Makrophagenprotein ist, das in einem Makrophagen exprimiert wird, und das zweite Immunzellprotein ein zweites Makrophagenprotein ist, das in einem Makrophagen exprimiert wird und sich von dem ersten Makrophagenprotein unterscheidet.

10. Immunfärbeverfahren nach einem der Ansprüche 1 bis 9, wobei das Zielprotein ein in einem Makrophagen exprimiertes Protein ist.

11. Immunfärbeverfahren nach einem der Ansprüche 1 bis 10, wobei das von dem Zielprotein abgeleitete Signal als eine Anzahl leuchtender Flecken gemessen wird, die von dem immunfluoreszenzgefärbten Zielprotein abgeleitet sind.

## Revendications

1. Méthode d'immunocoloration comprenant les étapes (A) à (D) suivantes :
une étape (A) de coloration d'une première protéine de cellule immunitaire exprimée dans une ou plusieurs cellules choisies parmi les macrophages, les cellules T, les cellules tueuses naturelles, les cellules dendritiques, les cellules B, les granulocytes et les plasmocytes ;
une étape (B) de coloration d'une seconde protéine de cellule immunitaire exprimée dans une ou plusieurs cellules choisies parmi les macrophages, les cellules T, les cellules tueuses naturelles, les cellules dendritiques, les cellules B, les granulocytes et les plasmocytes ;
une étape (C) de coloration d'une protéine cible ; et
une étape (D) consistant, après les étapes (A) à (C), à mesurer un signal dérivé de la protéine cible,
dans laquelle
les étapes (A) à (C) sont réalisées sur un même échantillon, l'échantillon étant une coupe de tissu prélevée sur un sujet ou des cellules obtenues par culture d'une cellule contenue dans un tissu prélevé sur un sujet, et la protéine cible étant au moins une protéine exprimée dans l'échantillon, et dans laquelle
la coloration dans les étapes (A) et (B) est une immunocoloration par colorant,
la coloration à l'étape (C) est une immunocoloration par immunofluorescence, et
l'immunocoloration par immunofluorescence à l'étape (C) est réalisée après les étapes (A) et (B).

2. Méthode d'immunocoloration selon la revendication 1, comprenant en outre une étape (E) d'identification d'une position et d'un nombre d'une cellule immunitaire sur la base de la coloration dans les étapes (A) et (B).

3. Méthode d'immunocoloration selon la revendication 1 ou 2, comprenant en outre une étape (F) d'identification d'informations sur un état d'expression de la protéine cible sur la base du signal dérivé de la protéine cible mesuré à l'étape (D).

4. Méthode d'immunocoloration selon la revendication 2 ou 3, comprenant en outre une étape (F) d'identification d'informations sur un état d'expression de la protéine cible sur la base de la position et du nombre de la cellule immunitaire identifiés à l'étape (E).

5. Méthode d'immunocoloration selon l'une quelconque des revendications 1 à 4, dans laquelle la première protéine de cellule immunitaire à l'étape (A) et la seconde protéine de cellule immunitaire à l'étape (B) sont colorées individuellement avec des colorants différents.

6. Méthode d'immunocoloration selon l'une quelconque des revendications 1 à 5, dans laquelle la première protéine de cellule immunitaire est colorée à l'étape (A) avec de la 3,3'-diaminobenzidine, et la seconde protéine de cellule immunitaire est colorée à l'étape (B) avec de l'Histogreen, et
l'étape (B) est réalisée après l'étape (A).

7. Méthode d'immunocoloration selon l'une quelconque des revendications 1 à 6, dans laquelle au moins une de la première protéine de cellule immunitaire et de la seconde protéine de cellule immunitaire comprend une protéine ayant au moins un ou plusieurs effets choisis parmi un effet stimulateur sur la réponse des cellules T et un effet antitumoral.

8. Méthode d'immunocoloration selon l'une quelconque des revendications 1 à 6, dans laquelle au moins une de la première protéine de cellule immunitaire et de la seconde protéine de cellule immunitaire est une protéine exprimée dans un macrophage.

9. Méthode d'immunocoloration selon la revendication 8, dans laquelle la première protéine de cellule immunitaire est une première protéine de macrophage qui est exprimée dans un macrophage, et la seconde protéine de cellule immunitaire est une seconde protéine de macrophage qui est exprimée dans un macrophage et est différente de la première protéine de macrophage.

10. Méthode d'immunocoloration selon l'une quelconque des revendications 1 à 9, dans laquelle la protéine cible est une protéine exprimée dans un macrophage.

11. Méthode d'immunocoloration selon l'une quelconque des revendications 1 à 10, dans laquelle le signal dérivé de la protéine cible est mesuré sous forme d'un nombre de spots luminescents dérivé de la protéine cible colorée par immunofluorescence.
